# EUROPEAN PATENT APPLICATION

(11) **EP 2 591 812 A1**
(43) Date of publication of application: **15.05.2013**
(21) Application number: 11188957.2
(22) Date of filing: 14.11.2011
(51) Int. Cl.: A61L 27/20, A61L 27/36

(54) **A dextran-based tissuelette containing platelet-rich plasma lysate for cartilage repair**

(71) Applicant: University of Twente, Institute for Biomedical Technology and Technical Medicine (MIRA), 7522 NB Enschede (NL)
(72) Inventor: Moreira Teixeira, Liliana Sofia, 7523 RG Enschede (NL); Dijkstra, Pieter Jelle, 7623 CP Borne (NL); Karperien, Hermanus Bernardus Johannes, 7151 DJ Eibergen (NL)
(74) Representative: Jansen, Cornelis Marinus

(57) **Abstract**

The invention relates to a dextran-based tissuelette comprising platelet-rich plasma lysate (PRPL), which can be used for tissue repair. In particular, the tissuelette of the present invention can be adapted to display properties similar to natural cartilage, while having increased chondrocyte-attraction. Also, it has a tuneable biostability that allows for degradation of the tissuelette material during natural tissue regeneration. In addition, the tissuelette displays full and covalent integration with the surrounding tissue when prepared directly at the site requiring tissue repair. The tissuelette of the present invention is primarily meant for use in cartilage repair, in particular cartilage repair upon degradation by osteoarthritis.

## Description

### Field of the invention

The invention relates to the field of tissue engineering. Particularly, the invention relates to a tissuelette that is formed by the crosslinking of polymers in the presence of platelet-rich plasma lysate. More particularly, the invention relates to the use of tyramine-functionalised dextran-based polymers in a tissuelette comprising platelet-rich plasma lysate, methods of producing such a tissuelette and uses thereof.

### Background of the invention

Cartilage is a natural connective tissue found in many parts of the body of higher animals, including humans. It is composed of an extracellular matrix comprising collagen (mainly type II), and further proteoglycans and, depending on the subtype, elastin. Chondrocytes are responsible for healing of cartilage damage, but because of the high stiffness and low penetration, they have little mobility inside natural cartilage and the self-regenerative capacity of cartilage is therefore low.

There are ample possibilities that can lead to cartilage damage, such as accidents, erroneous biomechanical positioning and age-related degeneration, among which osteoarthritis. Given the functional limitation and physical discomfort that accompanies cartilage damage such defects are considered a therapeutic and medical problem of high relevance. Unfortunately, cartilage has a limited potential for self-regeneration. Even for small defects self-regeneration is often not sufficient to ensure full timely regrowth and they predispose to early onset osteoarthritis. More severe cartilage defects usually defy full restoration, leaving the patient with disabilities and decreased quality of life. On top of that, the rising incidence of osteoarthritis due to an ageing population aggravates the need for viable cartilage repair strategies. Several approaches have been taken to date. In the case of cartilage damage resultant from osteoarthritis, pain management and total or partial prosthetic joint displacement are the most common therapies. Pain management, however, does not remove the underlying causes, and it also does not solve the motion impairment that may accompany cartilage defects. Total or partial joint displacement is usually performed only at the end stage of the disease, but mainly in elderly patients, because in younger patients, prosthetic replacement is associated with considerable problems that are due to the limited lifespan of existing implants. Therefore, regeneration of natural cartilage tissue is preferred.

One approach is provided by autologous chondrocyte transplantation (ACT). Chondrocytes are the only cell type found in cartilage, and are responsible for the natural production and maintenance of the cartilage matrix. They can be surgically harvested and expanded in vitro, so that in a second surgical procedure the chondrocytes can be introduced in the cartilage defect to promote natural cartilage growth. Alternatively, matrix-associated autologous chondrocyte transplantation (MACT) relies on a similar procedure, but expansion of harvested chondrocytes is performed in vitro using an artificial matrix mainly composed of collagen, which can then be introduced into the defect. Both procedures are highly invasive and cost intensive due to the need of at least two surgical procedures, and the requirement of in vitro tissue culture in the intermediate period. Apart from that, the cell isolation required for the initial chondrocyte expansion implies donor site damage, and the expansion is accompanied by chondrocyte dedifferentiation, resulting in decreased collagen type II and increased collagen type I expression.

Another approach for cartilage regeneration has relied on application of mesenchymal stem cells (MSC). Such cells, when isolated from for example bone marrow, adipose tissue or a wide variety of other tissues, are capable of chondrogenic differentiation under appropriate conditions. Although attractive in theory, methods for applying this approach in cartilage regeneration are still under investigation, and there is no common understanding as to what approach would be most appropriate. A major drawback that has surfaced in recent years is that the cells tend to diffuse away from the cartilage defect, rendering this an approach with low effectiveness, if at all. The alternative, growing cartilage in vitro from MSCs and use this to replace damaged cartilage in a surgical procedure leads to unevenly distributed cartilage tissue. Cartilage tissue engineering approaches therefore require a suitable scaffold to promote mechanical stability while at the same time provide an appropriate environment to facilitate the tissue repair process. The processes for production of such biocompatible materials reported to date can not be accompanied by inclusion of cells of whatever type due to the harsh conditions required for their formation. Recently however, biocompatible polymer systems have been reported that provide macromolecular networks that can be formed by crosslinking hydroxyphenyl groups in an enzymatic peroxidase reaction. In an approach using tyramine-functionalised hyaluronan (WO 2006/010066), peroxidase crosslinking is used to provide a biocompatible macromolecular network with tuneable properties to mimic a long range of human tissue, varying from cartilage to kidney. These hydrogels are meant as tissue replacement, among others also for bone or cartilage replacement (US 7368502). Although mention is made of the possibility to include living cells for varying purposes, the mechanical properties are aimed at replacing tissue, among which cartilage. Also, despite the possibility to crosslink these materials in situ, it is not clear to what extent they integrate with the natural tissue. Furthermore, as these materials are intended as tissue replacement strategies, sufficient regeneration of natural tissue is not achieved.

A different hydrogel, based on dextran-polymers crafted onto a hyaluronan backbone, has been described in WO 2011/059325, the contents of which are incorporated herein by reference. This gel is superior with respect to mechanical properties, biostability and tuneability, and has been shown to integrate well with cartilage defects. Moreover, inclusion of heparin-polymers into the structure promotes chondrocyte- or chondrocyte-progenitor diffusion into the structure, thereby promoting regeneration. In WO 2011/059326, the contents of which are incorporated herein by reference, this latter hydrogel mixture is described in detail.

In a different approach, direct injection with platelet-rich plasma (PRP) has been attempted to promote cartilage growth. Also, PRP can be gelatinized to result in a sort of hydrogel, which has some cartilage-regenerative properties. However, such gels suffer from high biodegradation and poor mechanical properties. In addition, there have been attempts to use alginate or gelatine, with or without combination with natural "glue", as carrier systems for bone or cartilage repair, which because of the main expression of collagen I present difficulties in cartilage regeneration.

Therefore, there currently is no all-round and reliable method for cartilage repair. Partial recoveries by expensive and invasive treatment schedules are state of the art in cartilage repair, and despite a broad range of possible treatments in varying stages of experimental development, there is currently no agreement as to how to arrive at a suitable method of therapy. Therefore there is a need for an improved and feasible treatment that will promote cartilage regeneration.

### Summary of the invention

The invention provides a tissuelette comprising a crosslinked tyramine-functionalised dextran-based polymer (also referred to as Dex-TA) and platelet-rich plasma lysate, the tissuelette having tuneable biostability and tissue-resembling mechanical properties and being seamlessly and covalently attached to the surrounding tissue, thereby promoting natural tissue growth so as to allow replacement of the tissuelette by natural tissue. In particular, the tissuelette comprises crosslinked tyramine-functionalised dextran-based polymers and platelet-rich plasma lysate (PRPL) for regeneration of natural cartilage. In one embodiment, the tissuelette is generated by in situ crosslinking of a solution comprising a tyramine-functionalised dextran-based polymer in the presence of PRPL, with a peroxidase enzyme in the presence of non-harmful amounts of hydrogen peroxide (Dex-TA hydrogel). In another embodiment, the tissuelette comprises a tyramine-functionalised dextran-based polymer crafted onto a hyaluronan backbone (HA-g-Dex-TA), in the presence of PRPL. In yet another embodiment, Dex-TA or HA-g-Dex-TA in the presence of PRPL is supplemented with an optimal amount of tyramine-functionalised heparin- or other (bio)polymers. The tissuelette provides mechanical properties resembling natural tissue, preferably cartilage, while at the same time natural cells, preferably chondrocytes, that can diffuse from the surrounding natural tissue or can be added during formation of the tissuelette, promote natural cell growth at the site, so that the tissuelette is replaced by or supplemented with natural tissue.

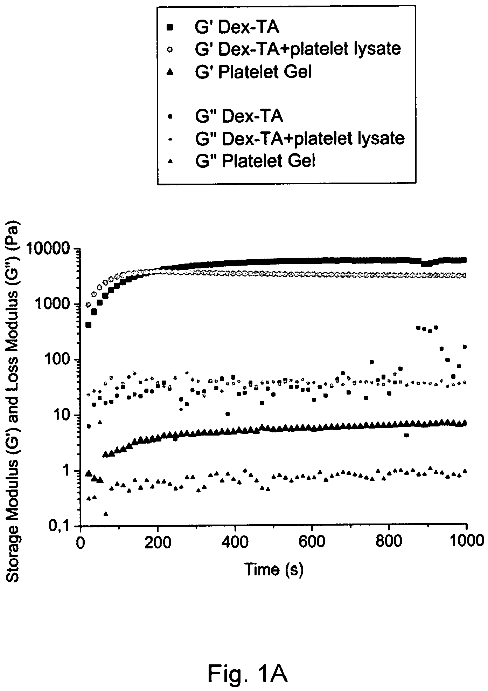

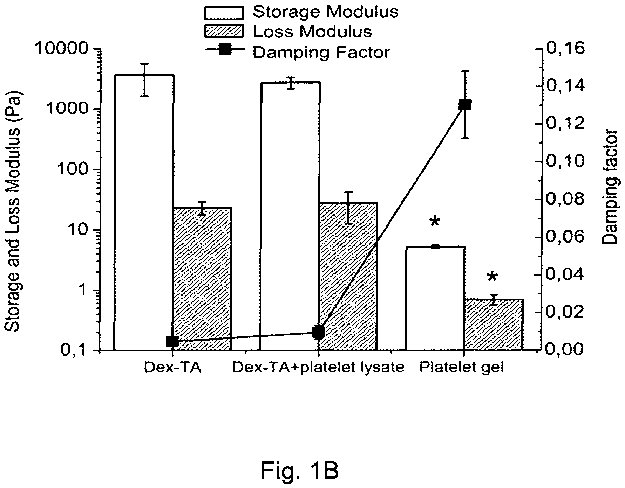

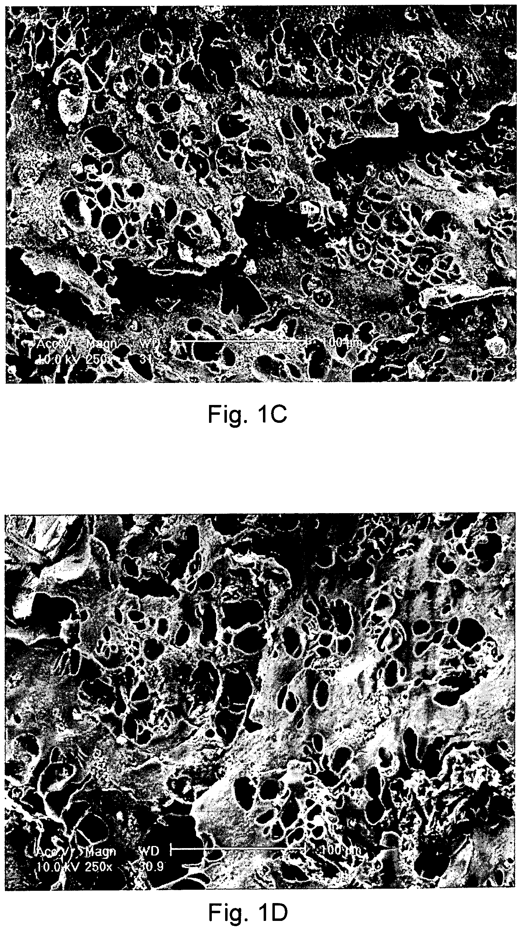

Description of drawings
*Figure 1**. Rheological analysis and hydrogel morphology.*
   A) Storage (G') and loss (G") modulus of crosslinked Dex-TA, crosslinked Dex-TA with platelet lysate and pure platelet gel, as a function of time; B) Storage and loss modulus plateau values, as well as damping factors (tgδ=G"/G') of crosslinked Dex-TA, crosslinked Dex-TA with platelet lysate and pure platelet gel; C) Representative SEM image of critical point dried crosslinked Dex-TA; D) Representative SEM image of critical point dried crosslinked Dex-TA with platelet lysate, showing no difference in porosity compared with crosslinked Dex-TA only. Scale bars correspond to 100 µm.

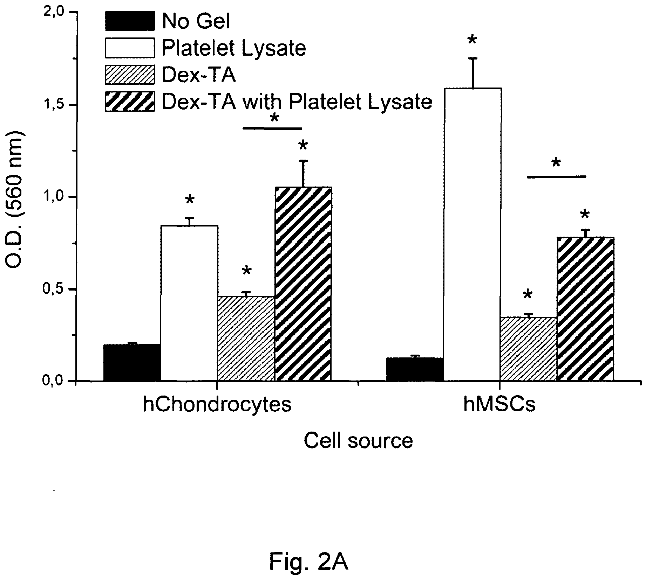

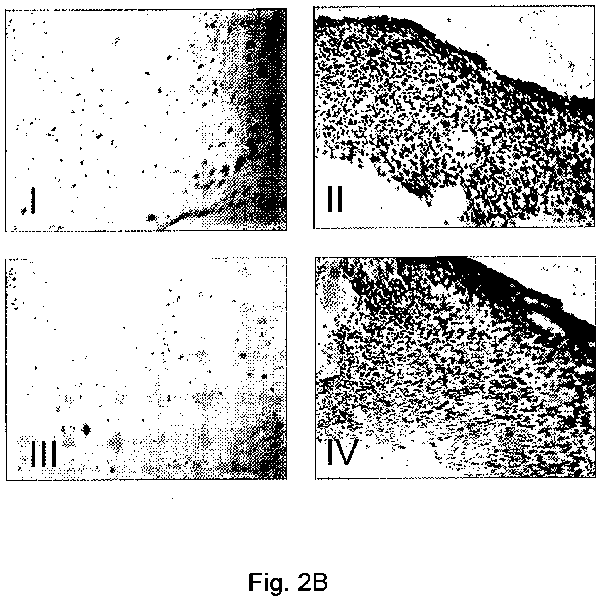

*Figure 2**. Platelet rich lysate renders Dex-TAgels chemo-attractant.*
   A) Cell migration was assessed by a 24-hours trans-well cell migration assay using either human chondrocytes (hChondrocytes) or human bone marrow derived mesenchymal stromal cells (hMSCs). Platelet lysate mixed in the culture medium, crosslinked Dex-TA and crosslinked Dex-TA with platelet lysate were prepared on the wells and the migratory cells that crossed the membrane were quantified. The platelet lysate as a medium component or incorporated in Dex-TA acted as a chemo-attractant. B) MTT assay performed in the gels immediately after the collection of the membranes for quantification. The metabolically active cells that crossed the membrane and adhered onto the gels are stained purple. I) Dex-TA with adhered hchondrocytes; II) Dex-TA with platelet lysate with adhered hchondrocytes; III) Dex-TA with adhered hMSCs; IV) Dex-TA with platelet lysate with adhered hMSCs.

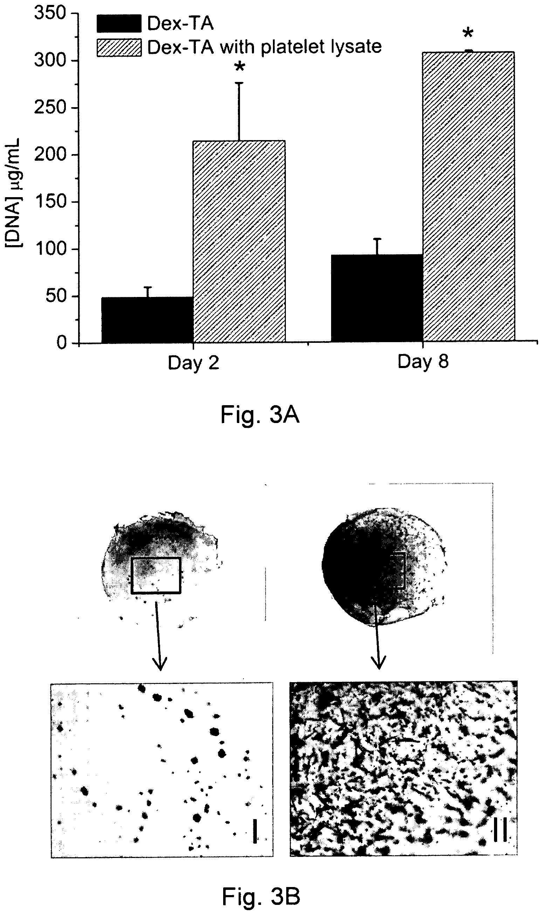

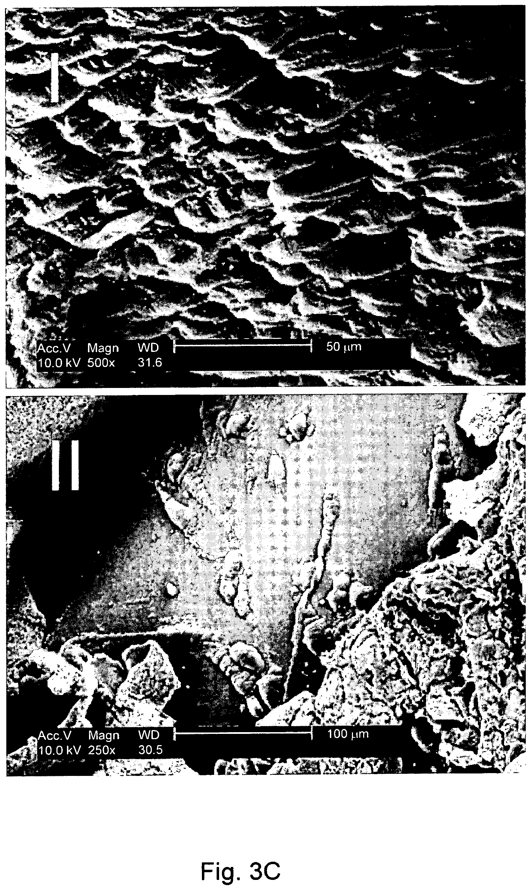

*Figure 3**. Adhesion and proliferation profile.*
   A) DNA quantification of MSCs that were seeded onto the crosslinked Dex-TA prepared with or without platelet lysate (2,5x10⁴ cells per cm²). The assays were performed after 2 and 8 days in culture. B) MTT assay of the MSCs adhered onto crosslinked Dex-TA prepared with or without platelet lysate, after 8 days in culture. Metabolically active cells are stained purple. C) Representative SEM image of critical point dried Dex-TA tissuelette with adhered MSCs, after 8 days in culture. I) Top view II) Cross-section, showing cells that have migrated from the top into the interior of the gel.

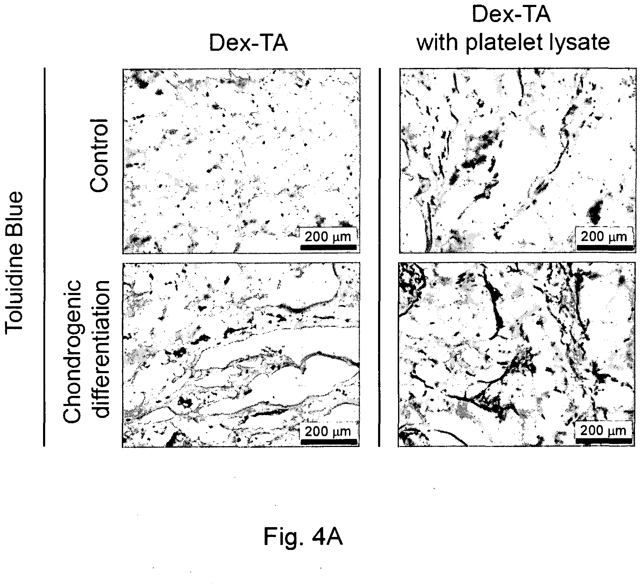

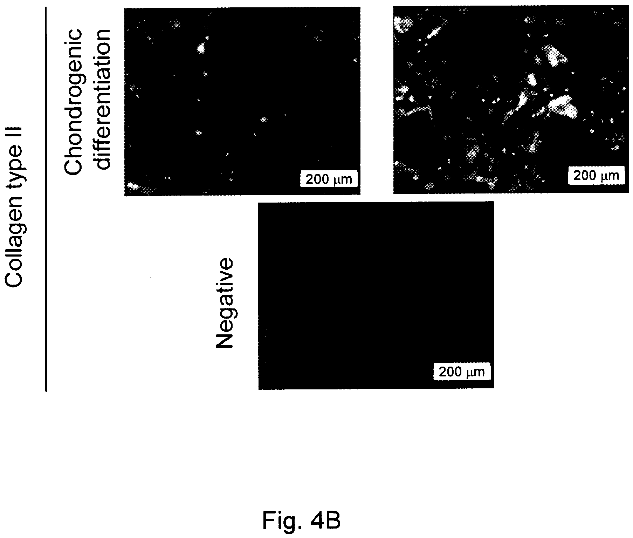

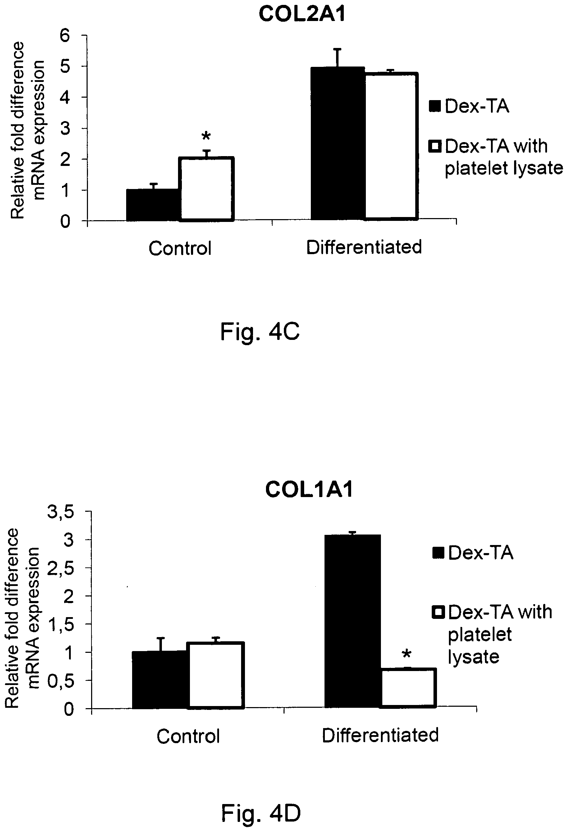

*Figure 4**. Chondrogenic differentiation.*
   A) Histological evaluation of representative samples of crosslinked Dex-TA with or without platelet lysate with incorporated MSCs, cultured for 21 days in expansion or chondrogenic media, stained with Toluidine blue (purple color indicates GAG deposition). B) Collagen type II immuno-fluorescence staining of representative samples of Dex-TA with or without platelet lysate with incorporated MSCs, cultured for 21 days in chondrogenic media (collagen type II is stained in green and cell nuclei are stained blue). The negative control sample was obtained by absence of incubation with primary anti-body. C) Expression levels of COL2A1 and COL1A1 genes analyzed by quantitative PCR of gel/cell constructs of Dex-TA with or without platelet lysate with incorporated MSCs cultured for 21 days in expansion or differentiation media. Gene expression was normalized to the expression of GAPDH.

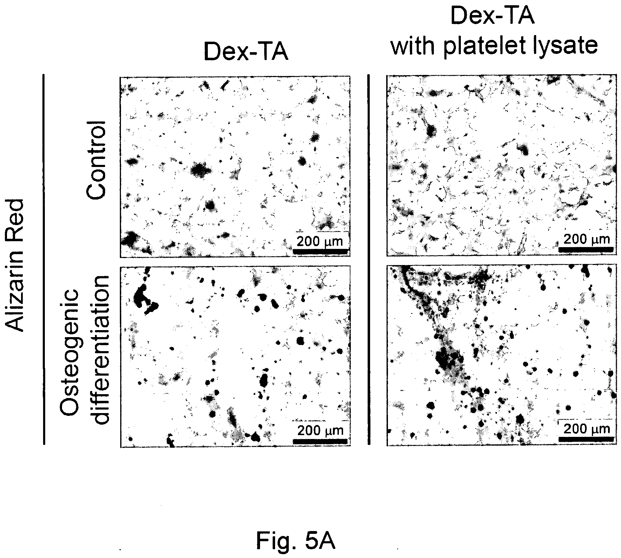

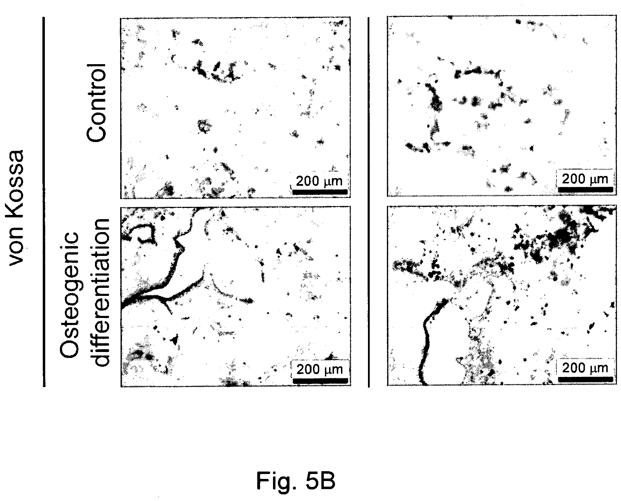

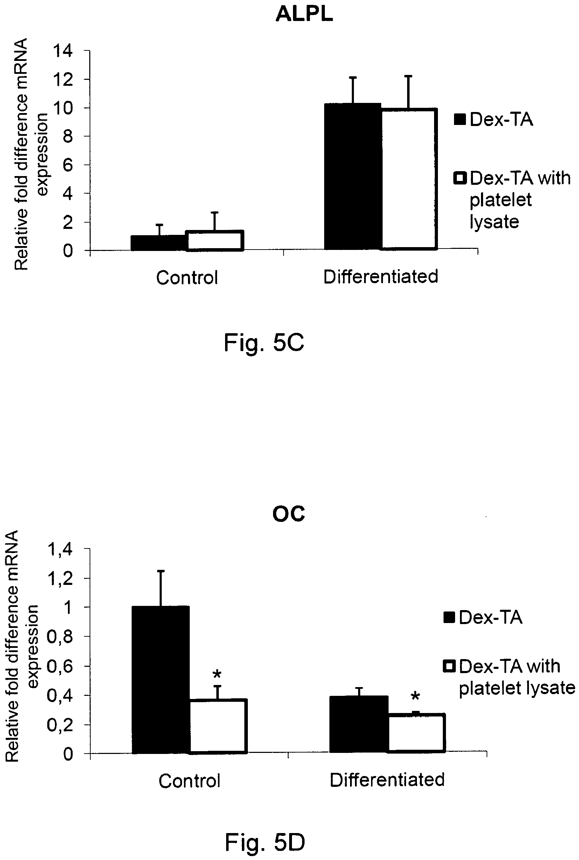

*Figure 5**. Osteogenic differentiation.*
   A) Histological evaluation of representative samples of Dex-TA with or without platelet lysate with incorporated MSCs, cultured for 21 days in expansion or osteogenic media, stained with Alizarin Red (red colour indicates calcium deposition). B) Histological evaluation of representative samples of Dex-TA with or without platelet lysate with incorporated MSCs, cultured for 21 days in expansion or osteogenic media, stained with von Kossa (dark brown color indicates mineralization). C/D) Expression levels of ALPL and OC genes analyzed by quantitative PCR of gel/cell constructs of Dex-TA with or without platelet lysate with incorporated MSCs cultured for 21 days in expansion or differentiation media. Gene expression was normalized to the expression of GAPDH (n=3).

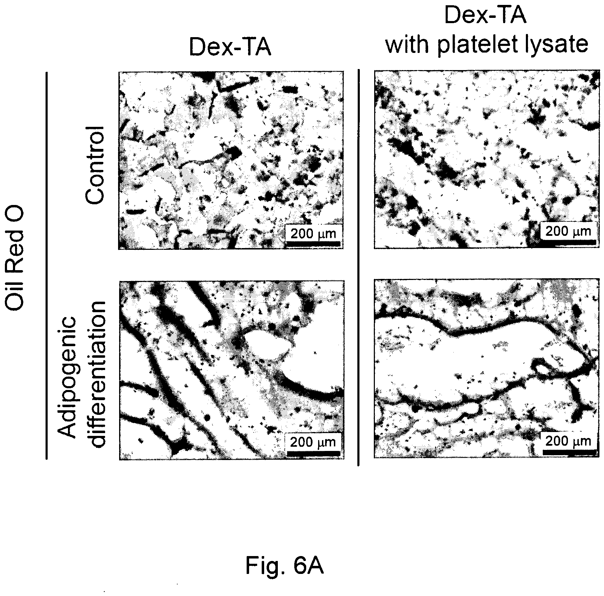

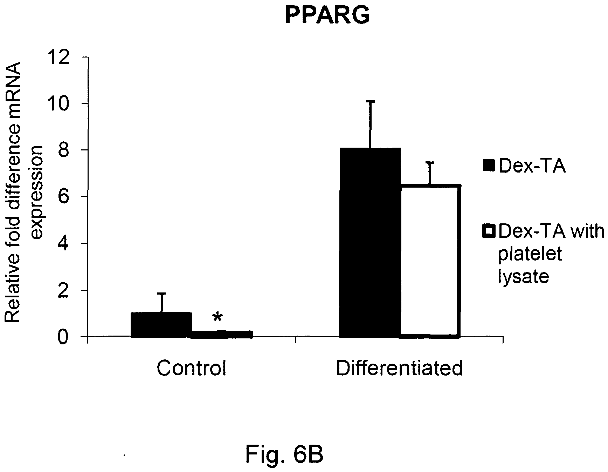

*Figure 6**. Adipogenic differentiation.*
   A) Histological evaluation of representative samples of Dex-TA with or without platelet lysate with incorporated MSCs, cultured for 21 days in expansion or adipogenic media, stained with Oil Red O (red colour indicates fat deposition). B) Expression levels of PPARG mRNA analyzed by quantitative PCR of gel/cell constructs of Dex-TA with or without platelet lysate with incorporated MSCs cultured for 21 days in expansion or differentiation media. Gene expression was normalized to the expression of GAPDH (n=3).

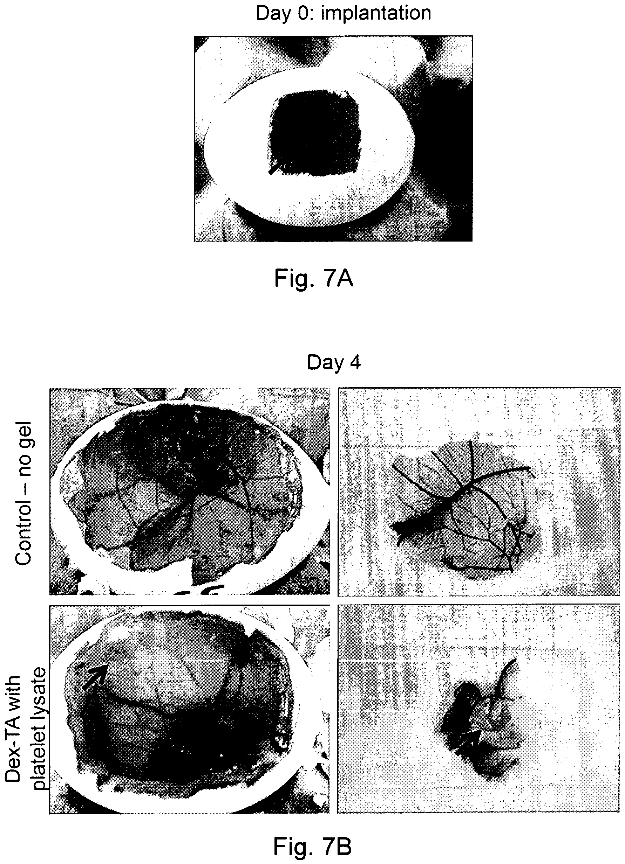

*Figure 7**. Vascularization potential using the chorioallantoic membrane (CAM) model.*
   A) After 7 days of incubation at 37 °C, crosslinked Dex-TA with platelet lysate were placed in contact with the CAM, sealed and returned to the incubator. B) Four days after placing the gels in contact with the membrane, images were collected to study vasculature development. After fixation, the gels with the CAM were excised and transferred onto glass slides. The control corresponds to normal membrane development without gel.

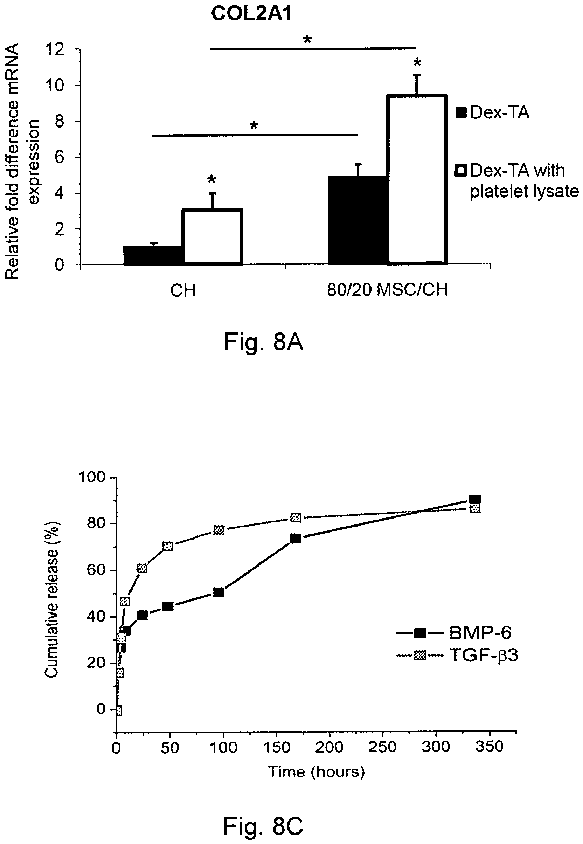

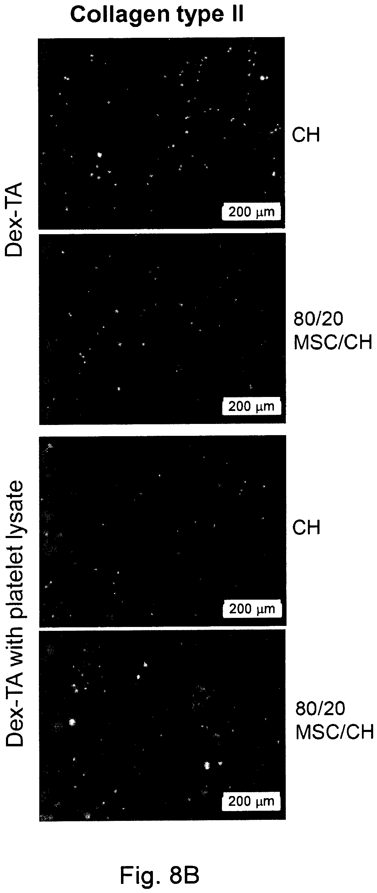

*Figure 8**. Platelet rich lysate enhances chondro -induction.*
   A) Expression levels of COL2A1 gene analyzed by quantitative PCR of gel/cell constructs of Dex-TA with or without platelet lysate with incorporated hChondrocytes or co-cultures of hMSCs with hChondrocytes (80/20 ratio), cultured for 21 days in chondrogenic medium without TGF-β3 supplementation. Gene expression was normalized to the expression of GAPDH (n=3). B) Collagen type II immuno-fluorescent staining of representative samples of Dex-TA with or without platelet lysate with incorporated hChondrocytes or co-cultures of hMSCs with hChondrocytes (80/20 ratio), cultured for 21 days in chondrogenic medium without TGF-β3 supplementation (collagen type II is stained in green and cell nuclei are stained blue). Scale bars correspond to 200 µm. C) Release profile of the TGF-β3 and BMP-6 present in the platelet lysate incorporated into Dex-TA tissuelettes, up to 14 days. Cumulative release of both growth factors was obtained by quantification of TGF-β3 and BMP-6 in the platelet lysate and was normalized to the total amount present in the lysate.

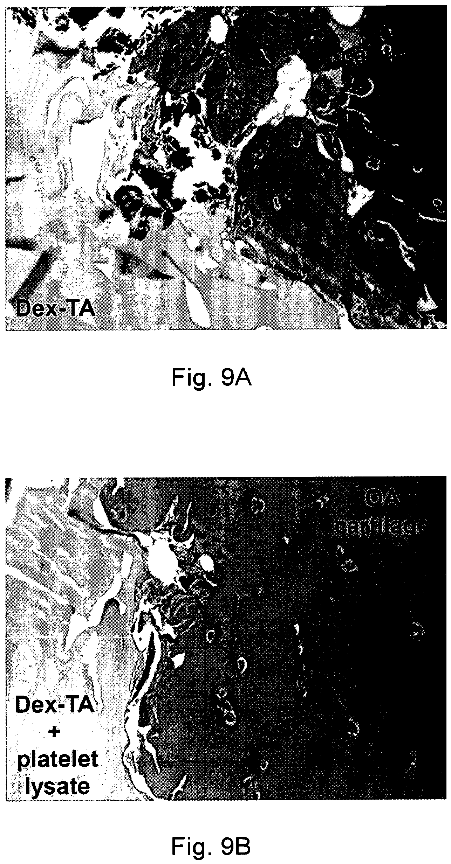

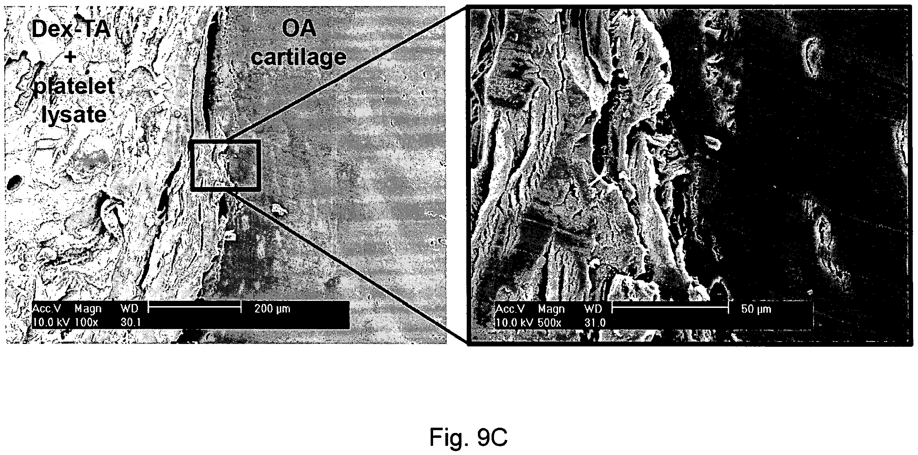

*Figure 9**. Interface tissuelette-OA cartilage.*
   The integration and adhesion between the crosslinked Dex-TA without (A) and with platelet lysate (B) onto standardized samples of human OA cartilage. Representative sections of the samples were stained with H&E. C) Representative SEM image of the interface between Dex-TA tissuelette with platelet lysate and OA affected cartilage. The intricate interaction between the tissuelette and the host tissue is evidenced, highlighting the absence of gaps at the interfacial area.

### Detailed description

A tissuelette, according to the invention, is a specific type of gel, preferably a hydrogel, with tissue-regenerating properties and tissue-resembling mechanical properties, that is seamlessly integrated with the surrounding tissue when formed in situ, and has tuneable biostability, and that may or may not comprise living cells. It is formed by injection of a tissuelette-forming composition comprising the elements tyramine-functionalised dextran-based polymers, PRPL and suitable solvents, to be crosslinked with hydrogen peroxide and peroxidase enzyme in suitable solvent(s), so that when combined they form the tissuelette.

A gel, according to the present invention, is a 3-dimensional macromolecular network comprising a crosslinked tyramine-substituted dextran-based polymer. Also, such gel can be referred to as a hydrogel, because of its high water content.

Biostability according to the present invention is a measure for the stability of a gel inside the living body. This entails both molecular stability against for instance degrading enzymes, and mechanical stability as presented by resistance against motility or friction in the location of presence. It can be determined by measuring degradation of the gel in vivo or in vitro. This can be done for instance by determining loss of (dry) weight of the gel in time, determining swelling ratio in time or determination in time of a different variable, in the presence of compounds or enzymes normally present in the location of application, in the presence of compounds added to the tissuelette during formation, or in the presence of stress or friction comparable to that present in the location of application.

In situ, as used herein, means that injection of the tissuelette-forming composition occurs into a site requiring tissue repair, and that formation of the tissuelette occurs by inducing the crosslinking process in that same location. The degree of substitution (DS) is the amount of tyramine units grafted per 100 disaccharide units, which can be measured by ¹H NMR spectroscopy by comparing the integral of the signal at δ 5.0 ppm (dextran anomeric protons) with the integrals of the signals at δ 6.5-7.5 ppm (tyramine aromatic protons).

The present invention relates to a tissuelette provided by a tissuelette-forming composition comprising platelet-rich plasma lysate and tyramine-functionalised dextran-based polymers, crosslinkable by enzymatic peroxidase in the presence of hydrogen peroxide, which can be used for regenerating cartilage tissue. A tissuelette according to the present invention is a hydrogel having properties, in particular mechanical properties and interaction with living tissue, resembling those of natural tissue, preferably cartilage tissue, that may or may not comprise living cells, preferably chondrocytes. A tissuelette according to the present invention is used as an artificial tissue that integrates into and has mechanical properties resembling the natural tissue, promoting regeneration of tissue beyond its natural regenerative capacity, and displaying degradation, so as to allow full or partial replacement of the tissuelette by natural tissue. In particular, a tissuelette according to the invention has a biostability that can be adapted to the intended use and environment in which it is to be deployed. The molecular weight, degree of substitution of tyramine units and the concentration of the tyramine-functionalised dextran-based polymer solution, as well as the inclusion of various types of other polymers, are variables of particular value for achieving such tuneable biostability. In a much preferred embodiment, the tissuelette displays properties similar to those of natural cartilage, and promotes cartilage regrowth. In this embodiment, the tissuelette features a high storage modulus, an advantageous swelling behaviour and an adequate stability. Also, the tissuelette according to the present invention has a short crosslinking time which can be adapted by varying the degree of substitution (DS). The tissuelette-forming composition is injected as a liquid and crosslinked in situ, which allows for seamless and covalent integration into the surrounding tissue, especially with collagen-rich tissues, such as cartilage. In addition, the tissuelette has the ability to attract cells, especially chondrocytes, chondrogenic precursor cells or migratory mesenchymal stem cells, from the surrounding tissue. The invention particularly relates to the use of platelet-rich plasma lysate in the composition for tissuelette formation.

The tyramine-substituted dextran-based polymers of the present invention (Dex-TA) can be prepared following the procedures described in WO 2011/059325. Shortly, this entails functionalizing dextran-polymers with tyramine moieties through reaction with nitrophenylchloroformate in dimethylformamide in the presence of lithium chloride and pyridine. Said dextran polymer preferably has a molecular weight between 5 and 80 kDa, more preferably between 10 and 80 kDa. The resulting nitrophenylchloroformate-functionalised dextran is then substituted with tyramine to result in tyramine-functionalised dextran. The resulting tyramine-functionalised dextran polymer preferably has a degree of substitution (DS) of tyramine residues between 5 and 25.

Subsequently, the tyramine-substituted dextran-based polymers are combined with a suitable solvent and platelet-rich plasma lysate (PRPL), and possibly other inclusions as described below, which is then crosslinked using peroxidase enzyme and hydrogen peroxide to result in the tissuelette. Tissuelettes for cartilage regeneration that have been crosslinked in situ display a higher shear modulus than those preformed and later combined with host cartilage. The difference corresponds to an increase in the adhesion strength, and this effect is stronger for tissuelettes displaying higher DS. Also, the storage modulus of tissuelettes increases with increasing DS. Therefore, tissuelettes with a high degree of substitution are mechanically stronger, and display a stronger adhesion to the host tissue.

In one embodiment of the present invention, a co-solution of Dex-TA with PRPL is injected into a site requiring cartilage repair, and crosslinked using a peroxidase enzyme in the presence of hydrogen peroxide as described below. This embodiment is preferred because it results in excellent mechanical properties, adequate swelling behaviour and degradation rates, as well as covalent attachment to collagen-rich tissues in one a step injection procedure. Furthermore, this composition attracts and stimulates ingrowth from cells out of the surrounding tissue and, by providing a suitable mechanically stable microenvironment, allows for efficient de novo cartilage matrix formation by these ingrowing cells. In another embodiment, Dex-TA is first crafted onto a backbone of hyaluronan as described in WO 2011/059325. Subsequently, a solution of said tyramine-functionalised dextran crafted onto hyaluronan (HAg-Dex-TA) is combined with PRPL, before being injected into a site requiring cartilage repair. Formation of the tissuelette occurs by co-supplementing peroxidase enzyme and hydrogen peroxide as described below. The advantages of this embodiment are that the cell-attracting and cell-adhesive properties of the invention are very high. Moreover, cell proliferation is stimulated and chondrogenic differentiation of MSCs is triggered to greater extent compared to the combination of Dex-TA and PRPL only. In this embodiment, the favourable mechanical properties and degradation properties of Dex-TA hydrogels are retained and are complemented with the bioinstructive properties of hyaluronan and the possibility of the cells to remodel the hydrogel macromolecule network by expressing the enzyme hyaluronidase. In combination with PRPL this results in better de novo tissue formation.

In one embodiment of a tissuelette with PRPL, comprising tyramine-functionalised dextran grafted onto a hyaluronan backbone, the molecular weight of dextran is between 10 and 20 kDa, preferably around 14 kDa, while the degree of substitution of tyramine residues is between 5 and 20, preferably between 10 and 15. An advantage of tissuelettes according to this embodiment is that they have excellent characteristics for attracting cells and enabling cell-mediated remodelling, as cells are able to produce hyaluronidase.

In another embodiment, said dextran has a molecular weight between 20 and 40 kDa, preferably around 31 kDa. It is preferred if the degree of substitution of tyramine residues is between 5 and 25, preferably around 10 kDa. An advantage of this embodiment is that it has high strength, and a molecular structure that is highly suitably for retaining molecules, such as those found in PRPL.

Because the dextran moieties provide high biostability to the tissuelette, it is understood that the invention pertains not only to Dex-TA-based tissuelettes or tissuelettes based on Dex-TA grafted onto hyaluronan as described above, but that also other biomolecules may be used as backbone to alter the properties of the tissuelette, especially the biostability. For embodiments where high biostability is required, tissuelettes with high DS and/or high dextran content are preferred. Tissuelettes with high DS are stronger, and display stronger adhesion to the host tissue. In contrast, where lower biostability is required to allow for regeneration and ultimate displacement of the tissuelette by natural tissue, the tissuelette is preferably adapted so that dextran be grafted onto hyaluronan or other biomolecular backbones, such as polyamines, polycarboxylates or polysaccharides as is known in the art. Also, inclusion of other tyramine-substituted biomolecules including but not limited to polycarboxylates, polyamines and polysaccharides, including among others tyramine-substituted heparin, tyramine-substituted chondroitin-sulphate, tyramine-substituted hyaluronan, tyramine-substituted keratin sulphate, tyramine-substituted heparin sulphate and native extracellular matrix proteins like collagen, fibronectin, laminin either or not substituted with tyramine-residues into the tissuelette-forming composition is possible for this purpose.

The tissuelette-forming composition comprising PRPL and polymer with peroxidase-crosslinkable properties according to the present invention can be prepared using any suitable and biologically acceptable solvent, as described in WO 2011/059325. Preferably, PBS-buffer or similar buffers are used, although there is no intention to limit the present invention to the use of only these buffers. The peroxidase-polymerisable dextran-polymers and -conjugates are present in the tissuelette-forming composition comprising PRPL in any concentration, preferably of 5-40 % w/v, and more preferably in a concentration of 10-20 % w/v. Also, PRPL is present in the tissuelette-forming composition in any concentration, preferably a concentration of 20-80 % v/v, more preferred in a concentration of 30-70 % v/v, and most preferred in a concentration of 40 % v/v. Other components improving the functionality of the tissuelette as described elsewhere can be included in the tissuelette-forming composition, and also other components required for the formation of the tissuelette (i.e. peroxidase enzyme and/or hydrogen peroxide) can be included in the tissuelette-forming composition.

Platelet-rich plasma (PRP) is a blood concentrate that contains among others platelets, white blood cells, growth factors, cytokines and other bioactive proteins. Studies involving PRP have shown varying effects on tissue healing. The use of autologous platelet-rich plasma decreases occurrence of problems commonly associated with blood use for therapeutic applications, such as avoidance of allergic reactions, and decreased risk of transferring infective diseases. Various means of obtaining lysates from platelet-rich plasma are known in the art, such as for example aphaeresis from whole blood, the use of an autologous transfusion blood cell separator, application of the tube method, and others. It is contemplated that in the present invention any such method can be used. However, platelet-rich plasma obtained by aphaeresis from whole blood is particularly preferred for use in the present invention, because it has particularly favourable properties for use in cartilage regeneration. Apart from that, the ease of use, the widespread availability and the compatibility with requirements for white blood cell-reduced blood components in therapeutic applications make that aphaeresis is of preferred value for the preparation of PRP for use in tissuelettes according to the present invention.

Lysates can be obtained from PRP preferably by freeze-thawing, but application of osmotic processes like plasmolysis or cytolysis, as well as enzymatic lysis and other means can also be used. Preferably freeze-thawing is used because it has been shown to result in PRPL that incorporates well into the tissuelette and increases effectiveness of growth factor activity and release with a simple procedure.

According to the present invention, a tissuelette comprises PRPL. In a preferred embodiment, a dextran-based polymer-solution with peroxidase-crosslinkable properties is mixed with PRPL, hydrogen peroxide and peroxidase enzyme to obtain a 20-80 % v/v solution, preferably 30-70 % v/v and most preferred is a solution of 40 % v/v of PRPL in the tissuelette after formation. Also, PRPL can be added in combination with other molecules, and with or without inclusion of the hydrogen peroxide and/or the peroxidase enzyme required for the hydrogelation process, as long as the concentration of PRPL in the tissuelette after formation is as defined above. The tyramine-functionalised dextran-based polymers included in the tissuelette are present in a concentration of 5-40 % w/v, preferably in a concentration of 10-20 % w/v and more preferably 10 % w/v.

It is to be understood that apart from tyramine-functionalised dextran-based polymers and PRPL, other components may be included in a tissuelette according to the present invention. These may include but are not limited to (bio) molecules and/or enzymes, natural and/or synthetic polymers either or not substituted with tyramine-residues and other components such as ionic substances or pharmaceutical compounds that might aid in the therapeutic process by for instance increasing cartilage formation or further improving mechanical properties, or retaining cartilaginous matrix properties and stability, changing the properties of the resulting tissuelette, changing the properties of the lysate or components thereof, or supply further therapeutic assistance. In particular, other cells such as chondrocytes, fibroblasts, osteoblasts, mesenchymal stem cells and/or stem cells (not from human embryos), tissue specific progenitor cells or substances, such as growth factors, proteins, enzymes, pharmaceutical compounds, antibodies, salts, (bio)-polymers and/or others can be included in a tissuelette according to the invention.

The tissuelette-forming composition can be crosslinked using peroxidase enzyme in the presence of hydrogen peroxide. As described in WO 2011/059325 for gels using the same tyramine-functionalised dextran-based polymer basis, this means that a solution of hydrogen peroxide with a concentration between 0.001-0.9 % v/v is supplied to the tissuelette-forming composition. In one embodiment, hydrogen peroxide is the sole component of this solution. In another, much preferred embodiment, hydrogen peroxide is supplied together with the peroxidase enzyme that is responsible for crosslinking. Also, it is to be understood that hydrogen peroxide as mentioned here refers to a system that in conjunction with peroxidase enzyme allows for crosslinking of the tyramine-functionalised dextran-based polymers to form a tissuelette. Therefore, not only hydrogen peroxide can be used in this respect, but also other radicals capable of the same, including oxygen radicals, are envisioned as embodiments of aspects of the present invention.

Similarly, and similar to what is described in WO 2011/059325, peroxidase is to be understood as any enzyme that is capable of inducing the crosslinking of tyramine units as present in this invention. In one preferred embodiment, horseradish peroxidase (HRP) is used to achieve crosslinking. However, other peroxidase enzymes exist that are capable of inducing similar reactivity, among which human peroxidase enzymes, and plant peroxidases, such as soybean peroxidase. It will be understood by the person skilled in the art that any such enzyme capable of inducing such reactivity can be considered for use in the present invention.

HRP or enzymes with similar functionality are to be applied to the tissuelette-forming composition in PBS or similar buffer in an amount between 30 and 300 units/ml. More preferably, the concentration of the peroxidase enzyme is between 50 and 250 purpurogallin units/ml, and even more preferably the concentration of peroxidase enzyme is between 150 and 200 units/ml. In a preferred embodiment, the peroxidase enzyme is combined with the tissuelette-forming composition as a solution of 0.01-1 mg/ml, preferable 0.6 mg/ml. A particularly preferred embodiment for high cytocompatability is obtained with 0.25 mg HRP per mmol phenol moieties, and a molar ratio of hydrogen peroxide to tyramine between 0.05 and 1, preferably between 0.1 and 0.3, and more preferably 0.2.

Another much preferred embodiment is provided by combination of the solutions of hydrogen peroxide and peroxidase enzyme just before use to result in a single solution with concentrations as described above. Preferably, the solution of peroxidase enzyme is combined with the peroxide solution just before dilution with the tissuelette-forming composition. This solution is then combined with the tissuelette-forming composition comprising tyramine-functionalised dextran-based polymers and PRPL, so that the solution with hydrogen peroxide and peroxidase enzyme makes up 5-40 % v/v of the total injected volume that forms the tissuelette. More preferred is a concentration of 10-30% of the final volume, and most preferred is a concentration of the combined solution of hydrogen peroxide and peroxidase enzyme in the tissuelette-forming composition of 20 % v/v. This means that the tissuelette-forming composition makes up 60-95 % v/v of the final tissuelette, preferably 70-90 % v/v, most preferably 80 % v/v. The concentration of peroxidase enzyme in the total injected volume is preferably between 0.002 and 1 mg/ml, more preferably 0.06 mg/ml.

Crosslinking-times of the tissuelette formation are similar to the gelation times as described for the hydrogels described in WO 2011/059325, and can be determined by the vial tilting method. Essentially, the time it takes to crosslink a solution to form a tissuelette is short, between 10 s and 2 min, and in large dependent on the degree of substitution of tyramine units and the polymer concentration in the final crosslinkable solution. Fast crosslinking is achieved with high degrees of substitution, so that in one particular embodiment, a crosslinking time of 10 s can be achieved using HA-g-Dex-TA with a degree of substitution between 15 and 25, preferably 20, at a polymer concentration between 8 and 12 % w/v, preferably 10 % w/v. Crosslinking times become longer with diminishing degree of substitution, and also with lower polymer concentration, so that the time to crosslink solutions according to the present invention can be tuned by the person skilled in the art to meet specific therapeutic needs.

A tissuelette-forming composition according to the invention can be injected at a site requiring cartilage regeneration. Such sites are defined by but not restricted to knee-, ankle-, finger-, toe-, neck- and other joints with cartilage damage, where cartilage damage may result from accidents, age-related degeneration, among which osteoarthritis, and erroneous biomechanical positioning. However, the current invention can be envisioned to be applied to other sites requiring cartilage regeneration, for instance by use in restorative or aesthetic surgery. Similarly, the invention is envisioned to be applicable in other forms of restorative or aesthetic tissue engineering approaches, for instance in bone- or soft tissue engineering, treatment of ulcers, skin tissue engineering, muscle engineering or ligament reconstruction.

The elements that will form the tissuelette can be supplied separately or in any combination. In one embodiment, solutions are injected separately so that mixing occurs in situ. In another embodiment, solutions are mixed shortly before injection takes place. However, in a much preferred embodiment, PRP-lysate is combined in a solution comprising tyramine-functionalised dextran-based polymer, with or without the presence of other tyramine-substituted polymers, and hydrogen peroxide is combined with peroxidase enzyme, such that both solutions are injected simultaneously and under proper mixing. In one such embodiment, the use of a specialized syringe with two compartments and a mixing chamber that ensures proper mixing of separate solutions during injection is highly preferred.

The strength of the tissuelette is to be adapted in relation to its envisioned purpose. The mechanical properties of a tissuelette, such as storage and loss modulus and porosity, are essentially the same as the mechanical properties of hydrogels formed with the same polymer basis, but without the special characteristics provided by the combination with PRPL. For the purpose of use in cartilage tissue, the swelling behaviour and shear modulus provide good parameters, as do the storage modulus G', the loss modulus G" and the damping factor. The swelling power is defined as the amount of water uptake achievable by the hydrogel in the dried form, and is determined by freeze-drying and weighing a freshly prepared gel, and then determining its weight at equilibrium in PBS buffer solution. The swelling power is then defined as the ratio of the difference between the wet weight and the dry weight, to the dry weight. Rheological parameters such as shear and loss modulus, 3-point bending, tension, compression, shear and storage modulus are well known to those skilled in the art, and can be determined with a rheometer or a dynamic mechanical analyzer.

The swelling power of the tissuelettes according to the present invention is lower with higher degree of substitution. Also, HA-g-Dex-TA has higher swelling power than Dex-Ta. Furthermore, a higher concentration of the dextran-based polymer solution during crosslinking results in a higher storage modulus (G'). However, the storage modulus of HA-g-Dex-TA (varying from 370 - 18000 Pa) is comparable in size to that of Dex-TA, and both are much higher than that of previously reported fibrin based gels for use in cartilage repair.

It has been established that the highest biostability of tissuelettes according to the present invention is achieved by using compositions comprising PRPL and essentially only Dex-TA with high degree of substitution. Tissuelettes comprising dextran grafted upon hyaluronan or other biopolymers have been shown to display lower biostability, when comparing equal degree of substitution and equal polymer concentration. However, these tissuelettes have improved tissue inducing properties or better cell attracting properties and better facilitate cell ingrowth and matrix remodelling. An advantage in this respect is that biostability, as well as other biological and mechanical properties, can be tuned to fit certain therapeutic needs with respect to tissue regeneration. Also, it is envisioned that inclusion of PRPL into a Dex-TA tissuelette according to the invention will lead to similar biostability as would be seen in a hydrogel of similar composition but not containing PRPL. Biostability can easily be determined by the person skilled in the art by a variety of methods, including but not limited to measurement of swelling ratio and (dry) gel mass in time, dry sample porosity, storage and loss modulus, damping factor, and can therefore routinely be optimised for the application at hand.

It has been established that tissuelettes according to the invention allow for seamless integration into cartilage, covalently bound by tyramine-tyrosine links to the surrounding collagen. The same has been found for other tissue types, such as muscle and fat pad (which, in contrast to cartilage, are both mainly composed of collagen I), although the adhesion between tissuelette and adipose-tissue (fat) was lower than for muscle or cartilage. Micro- and nanoimaging techniques such as wet- and HR-SEM and micro-Raman spectroscopy reveal that an interface exists between a tissuelette formed in situ and natural cartilage, which displays averaged properties of the natural cartilage and tissuelette. Additionally, Raman spectroscopy reveals covalent bonding in the interface between natural collagen tissue (collagens, polysaccharides and aminoacids, most notably tyrosine) and tyramine-functionalised dextran-based polymers from the tissuelette. Also, in vitro experiments with fluorescently labelled tyramide units reveal that the enzymatic crosslinking reaction as used for tissuelette formation is also capable of covalently immobilizing tyramide units on tyrosine residues present in extra cellular matrix proteins like collagens. This allows for appropriate tissuelette formation both in crevice- and hole-shaped defects, with smooth or irregularly-shaped surfaces, as well as for direct fixation of a layered tissuelette onto collagen-rich tissue or other tissues with abundant extra cellular matrixes containing collagens or proteins alike.

It is known in the art that crosslinked Dex-TA has low cell interaction, for which reason such gels do not display significant cartilage formation. Cell interaction is defined by the amount of cell migration (among which are, importantly, chondrocytes, chondrocyte precursor cells and migratory mesenchymal stem cells (MSCs)), from natural cartilage or other natural tissue or synthetic environments, into the gel. In case of cartilage, it can be measured by diffusion of chondrocytes or MSCs into the gel in cell migration assays as exemplified below. The present invention of formation of a tissuelette comprising Dex-TA and PRPL solves the problem of low native (progenitor) chondrocyte, synovial-cells or bone-marrow derived MSCs attraction of Dex-TA gels, because inclusion of PRPL conveys to such gels cell-attracting properties, to a degree surpassing that of even pure PRPL. In one preferred embodiment, the high strength and biostability of Dex-TA gels is used to provide these favourable properties to the tissuelette, by inclusion of PRPL while crosslinking Dex-TA. The resulting tissuelette has high cell-attracting properties as well as high strength, and displays biostability suited for cartilage regrowth. In another preferred embodiment, the chondrocyte attraction of a tissuelette comprising Dex-TA is increased by including varying amounts of tyramine-functionalised heparin, and further increased by the inclusion of PRPL. This embodiment is preferred for high cell migration. Dextran-grafted hyaluronan has even higher cell-attracting properties than Hep-TA, so that a tissuelette comprising HA-g-Dex-TA and PRPL has both high cell-attracting properties and high strength. In this embodiment, the tissue regeneration capacity is high, while also allowing for tuning of the strength and biostability of the tissuelette by altering the degree of substitution and polymer concentration.

For native cartilage to regenerate, mesenchymal stem cells (MSC) need to differentiate into chondrocytes. In an embodiment of the present invention comprising added MSCs, it has been found that both expression of collagen type II genes, and synthesis of collagen type II, are much increased relative to a gel of similar composition but without platelet-rich plasma lysate. Moreover, inclusion of PRPL in the tissuelette suppressed osteogenic and adipogenic differentiation, thereby repressing collagen type I expression. Inclusion of PRPL alone is enough to preferentially trigger differentiation of MSCs into chondrocytes, much aiding the cartilage regeneration process. Therefore, in one preferred embodiment of the present invention, both PRPL and MSCs are included in the tissuelette according to the present invention to promote cartilage regeneration. Alternatively, chondrocytes are attracted from the surrounding tissue by the chemo-attractant properties provided by PRPL. Another much preferred embodiment combines both approaches, and relies on the chemo-attractant properties of the tissuelette to attract MSCs from bone marrow directly, and inducing their differentiation into chondrocytes, whereby the MSCs migrate naturally into the tissuelette due to combination of the invention with sub-chondral drilling.

The formation of a tissuelette according to the invention will typically require at least two separate solutions, that are to be injected in an area requiring cartilage repair under due mixing. Accordingly, the invention can be embodied by a kit of parts comprising all or a subset of the following components: polymers comprising tyramine-functionalised dextran-based polymers, platelet-rich plasma lysate, peroxidase enzyme, hydrogen peroxide, appropriate dilutants such as PBS buffer and optionally other components as described herein to be possibly included in the tissuelette, such as cells, biomolecules, therapeutic substances, salts, and other polymers. These parts can be combined at the spot, or partial combinations of a subset of these components can be marketed, such as a polymer solution in an appropriate solvent to be diluted with PRPL, or a solution of hydrogen peroxide in an appropriate buffer. It is to be noted that a solution of hydrogen peroxide comprising peroxidase enzyme is not stable enough for long-term storage, so that combination of hydogen peroxide and peroxidase enzyme should occur just before use. Preferably, the tissuelette-forming composition can be marketed as a sterile solution comprising the polymers, which is to be diluted with autologous PRPL. Separately, a solution comprising hydrogen peroxide and peroxidase enzyme can be prepared and combined with the tissuelette-forming composition. In a much preferred embodiment, the invention is marketed in combination with a specialised syringe comprising two compartments and a mixing chamber, whereby simultaneous injection of the contents of both chambers results in mixing in the mixing chamber, so that the composition is well mixed when arriving at the site requiring tissue repair. In this embodiment, a solution comprising peroxidase enzyme and hydrogen peroxide is prepared at the moment of application and supplied in one compartment, and a composition in PBS buffer comprising the tyramine-substituted dextran-based polymer is supplied in the other compartment, so that dilution of the polymer with autologous PRPL can be performed before injection. However, it is understood by those skilled in the art that other means of marketing the invention are conceivable, which are not to be excluded.

Similarly, the invention pertains to a method of forming the tissuelette, comprising reacting a tyramine-functionalised dextran-based polymer with a peroxidase enzyme in the presence of hydrogen peroxide and platelet-rich plasma lysate.

The tissuelette can be used for use in tissue repair, preferably cartilage. Accordingly, a subject in need of cartilage repair because of accident or cartilage degenerative diseases, such as osteoarthritis, or otherwise is injected with the tissuelette-forming composition and hydrogen peroxide and peroxidase enzyme in suitable buffers(s) in the site requiring tissue repair. The amount used is sufficient to completely fill the defect site in between the surrounding tissue. The formed tissuelette attracts cells from the surrounding tissue, facilitates their ingrowth and stimulates de novo tissue formation by the in growing cells resulting in complete regeneration of the defect site. Alternatively, sites requiring repair in other tissue, such as skin lesions, bone lesions, muscle or kidney, can be injected with a tissuelette-forming composition according to the invention at the site requiring such repair, under addition of peroxidase enzyme and hydrogen peroxide before, during or after injection so as to induce crosslinking and ensure immobilization of the tissuelette at the defect site by covalent bonding to extracellular matrix proteins. Preferably, the tissuelette will form by crosslinking in situ, by separate addition of hydrogen peroxide and/or peroxidase enzyme during or after injection of the tissuelette-forming composition. However, because the crosslinking process will start at the moment that alle elements are combined, it should be noted that in a situation where mixing of the tyramine-functionalised dextran-based polymers comprising PRPL with hydrogen peroxide and peroxidase enzyme occurs before injection, the croslinling will have started already during injection. In this case, the tissuelette is being injected, and integration with the surrounding tissue will occur after injection due to the continuation of the crosslinking process.

The invention may be used alone or in combination with other therapies, as can be determined by those skilled in the art. The invention will now be illustrated by the following non-restrictive examples.

### EXAMPLES

### Materials and methods

### Platelet collection and cell sources

Human MSCs were isolated from fresh bone marrow aspirates, obtained after written consent. Briefly, aspirates were resuspended using a 20-gauge needle, plated at a density of 500,000 cells/cm² and cultured in MSC proliferation medium consisting of Alpha-MEM (α-MEM, Gibco), 10% heat-inactivated fetal bovine serum (Biowhittaker), 0.2 mM ascorbic acid (Sigma), 2 mM L-glutamine (Gibco), 100 U/ml penicillin with 100 mg/ml streptomycin (Gibco) and 1 ng/ml basic Fibroblast Growth Factor (Instruchemie). Cells were expanded and used in passage 2.

Chondrocytes were isolated from human articular cartilage, obtained after total joint replacement surgery. Cartilage pieces were digested using collagenase type II (Worthington). Isolated cells were seeded at 3000 per cm² and expanded in chondrocyte expansion medium containing DMEM (Gibco), 10% heat-inactivated fetal bovine serum (Biowhittaker), 1x non-essential amino acids (100x, Sigma), 0.2 mM ascorbic acid, 0.4 mM proline (Sigma-Aldrich), 1% penicillin and streptomycin. Cells were expanded and used in passage 2.

Human blood was collected from healthy volunteers, after written consent, and directly passed through an aphaeresis unit, which separates the platelets, returning back the rest of the blood. The platelet-rich fraction was immediately frozen until further use. Two freeze-thawing cycles were used to lyse the platelets.

### Mechanical properties

Dex-TA with a degree of substitution of 15, defined as the number of tyramine (TA) moieties grafted per 100 disaccharide units, was prepared as described previously (WO 2011/0059325) .Rheology experiments were performed on an Anton Paar Physica MCR 301 rheometer with flat plate geometry (20 mm diameter, 0.5 mm gap) in oscillating mode, at 37°C. 20% (w/v) Dex-TA polymer solution was mixed with pure platelet lysate to obtain 10% (w/v) polymer solution. As a control, instead of platelet lysate, the polymer was mixed with culture medium to obtain 10% (w/v) polymer solution. The crosslinking method was induced by mixing the polymer with horseradish peroxidase (HRP) and hydrogen peroxide (H₂O₂) to form a tissuelette. The reaction conditions were 0,25 mg of HRP per mmol of phenol groups and the molar ratio of H₂O₂/TA was 0,2. After the samples were applied to the rheometer, the upper plate was lowered to a measuring gap size of 0.5 mm. The measurement was started immediately after sample preparation. A layer of oil was added to prevent evaporation. A frequency of 0.5 Hz and a strain of 0.1% were applied in the analysis. The measurement was allowed to proceed until the storage modulus reached a plateau value.

### Cell migration, assay

A cell migration assay based on a trans-well system was performed. 25% (w/v) Dex-TA polymer solution was mixed with pure platelet lysate to obtain 12.5% (w/v) polymer solution. As a control, instead of platelet lysate, the polymer was mixed with culture medium to obtain 12.5% (w/v) polymer solution. The crosslinking was induced by mixing with a solution of HRP/H₂O₂. The final concentration of polymer in the hydrogel was of 10% (w/v) and the final concentration of platelet lysate was of 40% (v/v). The tissuelette and hydrogel were added to the bottom of the migration plates (Kit CytoSelect 24-well Cell Migration Assay 8 µm, Colorimetric format; CBA-100 Cell Biolabs, Inc.). A volume of 300 µL of a cell suspension of human chondrocytes or human MSCs, containing 2x10⁶ cells/ml in serum-free media, was added to each insert and incubated for 24 hours. Afterwards, the media was carefully aspirated from the inside of the insert. The interior of the inserts was swabbed to remove all non-migratory cells. The inserts were transferred to a clean well containing 400 µL of Cell Stain Solution and incubated for 10 minutes at room temperature. The stained inserts were washed several times and allowed to air dry. Each insert was transferred to an empty well and 200 µL of Extraction Solution were added. After 10 minutes of incubation, each sample was transferred to a 96-well microtiter plate and the OD 560nm was measured in a plate reader. After the assay, the samples were incubated with an MTT solution that allowed visualization of metabolically active cells. Briefly, the samples were incubated with chondrocytes proliferation medium or MSC proliferation medium with the MTT solution (10% of the total medium volume of the stock solution of 5 mg/mL, Gibco) for 2 hours at 37°C in a 5% CO₂ atmosphere incubator. MTT is a pale yellow substrate that when reduced by living cells to formazan turns dark purple. This process requires active mitochondria. Images were captured using a colour camera (Nikon SMZ-10A).

### DNA quantification

The bottom of 48 well plates was covered with 200 µL of Dex-TA hydrogel (10% w/v) with or without platelet lysate. MSCs were seeded onto the samples (2.5x10⁴ cells per cm²). The cells were cultured in MSC proliferation media. Quantification of total DNA was determined with the CyQuant DNA kit, according to the manufacturer's description (Molecular Probes, Eugene, Oregon, USA), using a fluorescent plate reader (emission: 520 nm; excitation: 480 nm) (Perkin-Elmer, Victor 3, USA). The standard curve for DNA analysis was generated with λ DNA included in the kit. The assays were performed after 2 and 8 days in culture.

### Scanning Electron Microscopy analyses

MSC adhesion on scaffolds was analyzed by SEM (n=3). After 8 days in culture, Dex-TA crosslinked samples with and without platelet lysate were fixated in 10% (v/v) buffered formalin overnight at 4°C. Specimens were then dehydrated using sequential ethanol series (70%, 80%, 90%, 96%, and 100% (v/v), 1 hour each, and critical point dried using a *Balzers CPD 030* machine. The samples were gold-sputtered for further observation by SEM operated at 10 kV accelerating voltage.

### Multi-lineage differentiation

Samples of 100 µL Dex-TA (10% w/v) with or without platelet lysate were prepared with 10x10⁶ cells/mL of passage 2 MSCs and crosslinking was induced by adding HRP and H₂O₂ (10 µL of HRP of 0,6 mg/mL stock solution and 10 µL H₂O₂ 0,3% stock solution). The constructs were cultured for 21 days in expansion medium, chondrogenic, osteogenic or adipogenic media. For chondrogenic differentiation, MSCs were cultured in chondrogenic medium composed of DMEM supplemented with 0,1 µM dexamethasone (Sigma), 50 mg/mL ascorbic acid, 100 µg/mL sodium pyruvate (Sigma), 40 mg/mL L-proline, 1% insulin-transferrin selenous acid-plus premix (BD Bioscience Pharmingen), 100U/mL penicillin, 100 mg/mL streptomycin, and 10 ng/mL Transforming Growth Factor (TGF)-β3 (R&D Systems). Osteogenic differentiation was induced by culturing constructs in medium composed of α-MEM with 10 nM dexamethasone, 5 mM b-glycerolphosphate (Sigma), and 50 mM ascorbic acid-2- phosphate. Adipogenic differentiation was induced by culturing constructs in α-MEM supplemented with 10% fetal bovine serum, 100U/ mL penicillin, 100 mg/mL streptomycin, 1.6 µM insulin, 0.25 µM dexamethasone, 0.5mM 1-methyl-3-isobutylxanthine, 3, 7-dihydro-1-methyl-3-(2-methylpropyl)-1H-purine-2,6-dione, 3-isobutyl-1-methyl-2,6(1H,3H)-purinedione (IBMX, Sigma-Aldrich) and 50 µM indomethacin.

### Histological evaluation

After 21 days in culture, samples were fixed in 10% buffered formalin for 1 hour. After washing with PBS, the samples were embedded in cryomatrix (Cryomatrix, Shandon). A cryo-microtome (Leica) was used to collect 10 µm sections onto gold-coated slides. The cryomatrix was washed off the slides by incubation in distilled water for 10 minutes. Afterwards, histology and immunohistochemistry were performed. Toluidine blue (Fluka, 0,1% in demi-water, 10 minutes incubation) was used to stain glycosaminoglycans (GAGs). For collagen type II immuno-staining, the samples were treated with 10 mM citric acid buffer for 10 minutes, and then were washed with PBS/BSA 1%. Col2A1 monoclonal antibody (Purified mouse immunoglobulin IgG1, clone 3H1-F9, Abnova) was diluted at 1:100 in PBS/BSA 1% and incubated overnight at room temperature. After the sections were washed twice for 5 min in PBS/BSA 1%, an incubation step with Alexa Fluor 488-Goat anti-Mouse IgG1 (Invitrogen, Molecular Probes, 1:1000) followed, for 1 hour. After extensive washes, the sections were incubated with VECTASHIELD Mounting Medium with DAPI (Vector Laboratories, Burlingame, CA) for 1 minute and mounted. Alizarin Red and von Kossa stainings were used for osteogenic differentiation, and Oil Red O was used to stain adipocytes . The slides were then washed, dehydrated, mounted and analyzed using bright field or fluorescence (FITC filter) microscopy (Nikon Eclipse).

### Gene expression analysis

Tissuelette/cell constructs cultured for 21 days in expansion or differentiation media were washed with PBS and lysed using Trizol reagent (Invitrogen, Carlsbad, CA). Total RNA was isolated using the Nucleospin RNA II kit (Bioke) according to manufacturer's instructions. The RNA yields were determined using the Nanodrop2000 (ND-1000 Spectrophotometer, Isogen LifeScience). Subsequently, cDNA was synthesized using the iScript Kit (BioRad) according to the manufacturer's recommendations. Expression levels of individual genes were analyzed by quantitative PCR (MiQ, Bio-rad), using the following forward and reverse primers: Collagen type I (*COL1A1*) Forward 5' GTCACCCACCGACCAAGAAACC 3' Reverse 5' AAGTCCAGGCTGTCCAGGGATG 3'; Collagen type II (*COL2A1*) Forward 5' CGTCCAGATGACCTTCCTACG 3' Reverse 5' TGAGCAGGGCCTTCTTGAG 3'; Alkaline phosphatase *(ALPL)* Forward 5' ACAAGCACTCCCACTTCATC 3' Reverse 5' TTCAGCTCGTACTGCATGTC 3'; Osteocalcin (*OC*) Forward 5' GGCAGCGAGGTAGTGAAGAG 3' Reverse 5 'GATGTGGTCAGCCAACTCGT 3'; Peroxisome proliferator-activated receptor gamma (*PPARG*) Forward 5' GATGTCTCATAATGCCATCAGGTT 3' Reverse 5' GGATTCAGCTGGTCGATATCACT 3'; Glyceraldehyde-3- phosphate dehydrogenase (*GAPDH*) Forward 5' CGCTCTCTGCTCCTCCTGTT 3' Reverse 5' CCATGGTGTCTGAGCGATGT 3'. Gene expression was normalized to the expression of GAPDH.

### Chorioallantoic membrane (CAM) model

Fertilized eggs were purchased from a local farm (Enschede, The Netherlands) and placed horizontally in an incubator at a temperature of 37°C with 75% humidity. After 3 days of incubation, the eggs were punctured and 3 mL of liquid was removed. A rectangle was sawed off the eggshell, the window covered with tape and the viable eggs with a developing membrane were placed back into the incubator. On day 7, a 10% (w/v) Dex-TA tissuelette with platelet lysate was placed in contact with the CAM. Eggs without tissuelette were considered as controls. Four days after placing the tissuelette in contact with the membrane, images were collected to study vasculature development. Additionally, after sacrificing the embryos by ethanol injection, the CAM with the tissuelette was fixated in 10% buffered formalin and processed for further visualization under the microscope.

### Analysis of interface morphology

Samples of 100 µL of 10% Dex-TA with and without platelet lysate were crosslinked onto standardized biopsies of human OA cartilage (0.5x0.5x0.1 mm). The use of human material was approved by a local medical ethical committee. OA cartilage was obtained from the knee joints of adult patients suffering from late stage OA undergoing an arthroplasty, after informed consent. After fixation in 10% buffered formalin, the tissuelette-cartilage constructs were dehydrated and lyophilized (CPD030 Balzers Critical Point Dryer). Dried samples were sputtered with gold (Cressington sputter coater) and visualized using SEM, as described above. For histological analysis, the samples were dehydrated and embedded in paraffin. Sections of 5 µm were used for Haematoxylin and Eosin staining (H&E Sigma). Slides were assembled with resinous medium for visualization using light microscopy (Nikon Eclipse).

### Statistical analysis

Each experiment was performed in triplicate unless otherwise specified. The results are presented as mean ± standard deviation (SD). Experimental data were analyzed for statistical significance using a student t-test. Statistical significance was set to p-value ≤ 0.05 (*).

### Example 1

Experiments were performed with samples in which the polymer was dissolved in platelet lysate prior to peroxidase mediated crosslinking. Using this approach, the platelet lysate was homogeneously dispersed and successfully incorporated into crosslinked Dex-TA. Moreover, the crosslinking occurred within 1 minute and was not affected by dissolving the polymer in platelet lysate instead of PBS or culture medium.

The mechanical properties of 10% (w/v) Dex-TA tissuelettes incorporating platelet lysate were analyzed by oscillatory rheology and compared with crosslinked Dex-TA dissolved in culture medium, and with a platelet gel formed by mixing fibrinogen with thrombin in a 1:1 1 ratio. The kinetics of the gelation were evaluated by monitoring of storage and loss modulus throughout time. A gradual increase in the storage modulus was observed in all gel types, which reached a plateau within 5 minutes after starting the crosslinking reaction (Fig. 1a). The plateau moduli values of the gels were collected at 10 minutes and are presented in figure 1-B as the average of three measurements for each gel type. No significant differences were detected in the storage modulus of a Dex-TA hydrogel prepared in culture medium or in platelet lysate. The platelet gel showed very low mechanical properties, compared to the Dex-TA-based gels, yet it showed higher visco-elasticity, as indicated by a high damping factor value (defined as damping modulus/storage modulus). No differences were detected in the porosity of Dex-TA dissolved in culture medium or dissolved in platelet lysate, as shown in figures 1-C and 1-D, respectively.

### Example 2

The chemo-attractant properties of a 10% Dex-TA hydrogel without platelet lysate are poor and lower compared to a pure platelet lysate hydrogel (Fig 2-A), irrespective of the cell source. Mixing platelet lysate into a 10% Dex-TA solution and subsequent crosslinking considerably improved the chemo-attractant properties which surpassed the chemo-attractant properties of a pure platelet lysate hydrogel when primary chondrocytes were used. The metabolic activity of the cells that migrated and adhered onto the gels was assessed by MTT staining (figure 2-B). Using this assay, the cells that crossed the membrane and entered the gels were visualized. A limited amount of cells adhered onto Dex-TA gels without PRPL (Figure 2B, I and II). In contrast, a considerably higher amount of cells could be detected in Dex-TA gels mixed with platelet lysate, using both human chondrocytes and MSCs (Figure 2B, II and IV).

### Example 3

MSCs incorporated in Dex-TA gels in the presence or absence of platelet lysate were cultured in chondrogenic, osteogenic and adipogenic differentiation inducing medium. Expansion medium, without growth factors, was used as control. Both histological evaluation and gene expression analyses were performed. Cells showed the potential to differentiate into the different mesodermal lineages. The potential to differentiate into chondrocytes is indicated by the Toluidine Blue and Collagen type II staining. Both staining were more intense when cells were incorporated into Dex-TA gels with platelet lysate, as shown in figure 4-A. Platelet lysate did not have a beneficial effect on collagen type II mRNA expression when constructs were cultured in chondrogenic differentiation medium (Figure 4-C). In control medium, collagen type II mRNA expression was higher in the presence of platelet lysate. Interestingly, the presence of platelet lysate potently inhibited collagen type I mRNA expression (figure 4-D). Osteogenic differentiation can be detected using Alizarin Red staining, were calcium depositions stain red, and von Kossa staining, which stains mineralized areas dark brown. Both stainings appeared to be more intense when incorporating MSCs into Dex-TA with platelet lysate (figure 5-A). Yet no significant differences were found in ALP gene expression, which is an early osteogenic marker. The expression of OC, which is a late osteogenic marker, was decreased (figures 5-B and 5-D, respectively). This decrease was observed in control conditions and in osteogenic culture medium. No significant differences in adipogenic differentiation were observed between Dex-TA or Dex-TA-platelet lysate gels using Oil Red O staining and PPARg mRNA expression (figures 6-A and 6-B, respectively). Finally, to study the effect of platelet lysate on attracting blood vessel in growth into Dex-TA gels a CAM assay was performed. Visual inspection of the CAM, four days after placing the gels in contact with the membrane, did not reveal an effect of platelet lysate on blood vessel growth towards the gel construct (figure 7-B).

### Example 4

It is known in the art that combining MSC with primary human chondrocytes potentiates cartilage formation in micromass cultures. The test whether this potentiating effect is retained in Dex-TA hydrogels, we cultured human chondrocytes or MSCS only or a combination of 80% human MSCs mixed with 20% human chondrocytes in Dex-TA dissolved in culture medium or in platelet lysate. The constructs were cultured in chondrogenic medium without TGF-β3. Both Collagen type II gene expression and synthesis were significantly higher in presence of the platelet lysate in both culture systems, as shown in figures 8-A and 8-B. This increase was more dramatic, up to 9-fold, in a co-culture system in combination with platelet lysate.

### Example 5

To investigate whether improved chondrogenesis was due to an enrichment in growth factors associated with chondrogenic differentiation, such as TGF-β3 and BMP-6, in platelet lysate containing gels, we studied their release profile. Dex-TA hydrogels with platelet lysate were prepared similarly to the cell-gel constructs and the cumulative release was determined and expressed as percentage of the total initial content (figure 8-C). Notably, a burst release of both molecules from the gel was detected in the first days; however, approximately 20% of both growth factors remained entrapped within the gel network up to 14 days. The release of BMP-6 showed a more sustained profile, whereas TGF-β3 had a faster release, up to 70% in 4 days. At day 14, both growth factors had been released to the same extent, approximately 80%.

### Example 6

The peroxidase crosslinking reaction results not only in crosslinking of tyramine residues resulting in gel formation but also in tyramine-tyrosine cross links. The latter cross links enable direct fixation of the gel at collagen rich tissues. To test whether the incorporation of platelet lysate affected the fixation of Dex-TA gels at the cartilage surface, we assessed the morphology of the gel-cartilage interface. The gel integration at the defect area was simulated by gelating Dex-TA dissolved in culture medium or in platelet lysate on top of human OA cartilage samples. The cartilage-gel interface was evaluated by histology and SEM. As shown in figures 9-A and 9-B both gels were properly integrated with the cartilage. They were in close proximity with the defective cartilage surface, with only minor gaps at the interfacial area due to the extreme roughness of the fissures in the cartilage which are typical for OA. Similar results were obtained using SEM by which the interfacial area of Dex-TA with platelet lysate gels and cartilage could be visualized in more detail (figure 9-C). The gel showed close interaction with the cartilage specimen, most likely mediated by covalent bonds between the tyramine residues of the hydrogel and tyrosine residues present in collagen or other matrix proteins present in the cartilage tissue.

## Claims

1. A tissuelette comprising a crosslinked tyramine-functionalised dextran-based polymer and platelet-rich plasma lysate.

2. A tissuelette according to claim 1, wherein the tyramine-functionalised dextran-based polymer is covalently attached to a hyaluronan backbone.

3. A tissuelette according to claim 1 or 2, wherein the tyramine-functionalised dextran-based polymer has a molecular weight between 5 and 80 kDa.

4. A tissuelette according to any of the preceding claims, wherein the tyramine-functionalised dextran-based polymer has a degree of substitution of tyramine units between 5 and 25.

5. A tissuelette according to any of the preceding claims comprising a second tyramine-functionalised polymer, preferably a tyramine-functionalised heparin- or hyaluronan polymer.

6. A tissuelette according to any of the preceding claims, wherein the platelet-rich plasma lysate is autologous.

7. A tissuelette according to any of the preceding claims, wherein the platelet-rich plasma lysate is present in a concentration of 20 - 80 % v/v, more preferably 30-70 % v/v, and most preferably 40 % v/v.

8. A tissuelette according to any of the preceding claims, wherein the tyramine-functionalised dextran-based polymers are present in a concentration of 5-40 % w/v, preferably 10-20 % w/v, more preferably 10 % w/v.

9. A tissuelette-forming composition comprising platelet-rich plasma lysate and a tyramine-functionalised dextran-based polymer, crosslinkable by enzymatic peroxidase in the presence of hydrogen peroxide to obtain a tissuelette according to any of the preceding claims.

10. A tissuelette-forming composition according to claim 9 wherein the PRPL is present in a concentration of 20 - 80 % v/v, more preferably 30-70 % v/v, and most preferably 40 % v/v, and wherein the tyramine-functionalised dextran-based polymers are present in a concentration of 5-40 % w/v, preferably 10-20 % w/v, more preferably 10 % w/v.

11. A kit of parts comprising platelet-rich plasma lysate, a tyramine-functionalised dextran-based polymer, a peroxidase enzyme, hydrogen peroxide and a suitable solvent, for producing a tissuelette according to any of claims 1-8.

12. A kit of parts according to claim 11, wherein the peroxidase is horseradish peroxidase, preferably present in a concentration between 0.01 and 1 mg/ml, preferably 0.6 mg/ml, in said suitable solvent.

13. A kit of parts according to claims 11-12, wherein the hydrogen peroxide is present in a concentration between 0.001 and 0.9 % v/v in said suitable solvent, preferably 0.3 % v/v, in said suitable solvent.

14. A method of preparing a tissuelette comprising reacting a tyramine-functionalised dextran-based polymer with a peroxidase enzyme in the presence of hydrogen peroxide and platelet-rich plasma lysate.

15. A method of treating a subject in need of tissue regeneration comprising
- injecting a therapeutically effective amount of a tissuelette according to any of the claims 1-8 into a tissue defect in said subject, or
- injecting a first solution comprising a tyramine-functionalised dextran-based polymer and a second solution comprising a peroxidase and hydrogen peroxide (simultaneously) into a tissue defect in said subject, wherein the first and/or the second solution further comprises platelet-rich plasma lysate.
